(19) Europäisches Patentamt
European Patent Office
Office européen des brevets

(11) Publication number: 0 114 067 B1

(12) EUROPEAN PATENT SPECIFICATION

(45) Date of publication of patent specification: 29.04.87

(51) Int. Cl.[4]: C 07 K 5/06, C 07 C 83/10, C 07 D 295/08, A 61 K 37/02

(21) Application number: 84100300.7

(22) Date of filing: 12.01.84

# (54) Compounds for treating hypertension.

(30) Priority: 12.01.83 US 457611

(43) Date of publication of application: 25.07.84 Bulletin 84/30

(45) Publication of the grant of the patent: 29.04.87 Bulletin 87/18

(84) Designated Contracting States: AT BE CH DE FR GB IT LI LU NL SE

(56) References cited:
EP-A-0 012 401
DE-B-1 916 481
FR-M- 1 904
US-A-4 156 739

(73) Proprietor: USV PHARMACEUTICAL CORPORATION
1 Scarsdale Road
Tuckahoe New York (US)

(72) Inventor: Huang, Fu-chih
611 Knoll Road
Boonton New Jersey (US)
Inventor: Jones, Howard
79 Briarcliff Woods
Ossining New York (US)
Inventor: Lin, Clara J.
161 Moorland Drive
Scarsdale New York (US)
Inventor: Loev, Bernard
42 Penny Lane
Scarsdale New York (US)

(74) Representative: Patentanwälte Grünecker, Dr. Kinkeldey, Dr. Stockmair, Dr. Schumann, Jakob, Dr. Bezold, Meister, Hilgers, Dr. Meyer-Plath
Maximilianstrasse 58
D-8000 München 22 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 114 067 B1

Courier Press, Leamington Spa, England.

**Description**

This application relates to compounds, their pharmaceutically-acceptable salts, and pharmaceutical preparations made therefrom, having biological utility in the treatment of hypertension in subjects suffering therefrom.

Broadly stated, the present invention comprises compounds of the formula

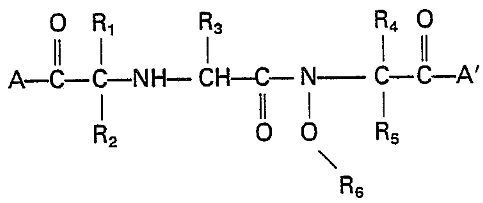

(1)

and their pharmaceutically-acceptable salts, wherein

A and A' are independently hydroxy, alkoxy, aryloxy, or hydroxyamino;

$R_1$, $R_2$, $R_4$ and $R_5$ are independently hydrogen, alkyl, aryl, aralkyl, fused cycloalkylaryl, fused arylcycloalkyl, aryloxyalkyl, or arylalkyloxyalkyl;

$R_3$ is hydrogen, lower alkyl, lower alkenyl, lower alkynyl, aryl, fused cycloalkylaryl, fused arylcycloalkyl, fused cycloalkylarylalkyl, fused arylcycloalkylalkyl, aralkyl, cycloalkyl, or heterocyclic; and

$R_6$ is hydrogen, alkyl, alkenyl, alkynyl, acyl, cycloalkyl, cycloalkylalkyl, polycycloalkyl, polycycloalkylalkyl, aryl, aralkyl, polycyclic aryl, fused cycloalkylaryl, fused arylcycloalkyl, fused arylcycloalkylalkyl, or fused cycloalkylarylalkyl;

wherein the alkyl, alkoxy, alkenoxy, alkenyl, and alkynyl groups may be substituted with hydroxy, acyloxy, aryl, alkoxy, aryloxy, amino, mono- or dialkylamino, acylamino, mercapto, mercaptoalkyl, or alkylthio; the aryl and cycloalkyl rings may contain one or more hetero atoms, and may be substituted with carboxylic acid, cyano, carbolower alkoxy, alkyl, hydroxy, alkoxy, hydroxyalkyl, halo, haloalkyl, mercapto mercaptoalkyl, alkylthio, amino, alkylamino, aminoalkyl, nitro, methylenedioxy, or sulfamyl groups, and the cycloalkyl groups may be saturated or unsaturated.

Preferred compounds of the present invention are those of the general formula given above in which A and A' are independently hydroxy or lower alkoxy; $R_1$, $R_2$, $R_3$, $R_4$ and $R_5$ are each hydrogen, alkyl, aryl, aralkyl, or w-aminoalkyl wherein the amino is mono- or disubstituted with hydrogen, alkyl, aryl, or aralkyl, or is incorporated in a saturated or unsaturated one- or two-ring heterocyclic moiety containing preferably up to 12 atoms in the ring; and

$R_6$ is hydrogen, alkyl, aryl, aralkyl, cycloalkyl, alkoxyalkyl, aminoalkyl, heteroaryl, heteroaralkyl, or fused arylaryl, any of which can be substituted or unsubstituted. Included as preferred groups are groups in which $R_6$ provides diuretic activity to the compound (1), e.g., sulfamyl-chloro-phenyl.

The alkyl groups per se and the alkyl moieties in alkoxy, aralkyl, cycloalkyl, aminoalkyl may be straight-chained or branched and preferably contain from 1 to 9 carbon atoms. Such groups include methyl, ethyl, propyl, isopropyl, butyl, isobutyl, tertiary-butyl, amyl, iso-amyl, hexyl, octyl. Preferably the alkyl groups are lower alkyl, which term shall refer to alkyl groups containing from 1 to 6 carbon atoms, straight-chained or branched.

The alkenyl and alkynyl groups and moieties can also be straight or branched-chained groups containing from 2 to 9, and preferably 2 to 6, carbon atoms. Such groups include vinyl, ethynyl, propenyl, isopropenyl.

The acyl groups include such groups as alkanoyl, aroyl, and aralkanoyl, wherein the alkyl and aryl moieties are defined herein, as well as sulfonyl, sulfamoyl, carbamoyl, optionally containing and alkyl moiety with 1 to 9, and preferably 1 to 6, carbon atoms.

The preferred substituents on the above alkyl, alkenyl, alkynyl, and acyl groups include hydroxy, alkoxy, amino, alkylamino, dialkylamino, mercapto, alkylmercapto.

The cycloalkyl groups and moieties are saturated or unsaturated and preferably contain 3 to 9 carbon atoms. By "polycycloalkyl" is meant 2 or more fused cycloalkyl rings, having a total of up to 20 carbon atoms. The cycloalkyl, polycycloalkyl, polycyclic aryl, and fused arylcycloalkyl structures can also contain one or two hetero atoms, i.e., a sulfur, oxygen, or nitrogen atom, thereby forming a hetero-ring.

Preferred cyclic and polycyclic rings structures include such radicals as cyclopropyl, cyclobutyl, cyclopentyl, cyclohexyl, cycloheptyl, adamantyl, norbornyl, phenyl, tolyl, benzyl, phenethyl, indolyl, dimethoxyphenyl, hydroxybenzyl, indanyl, naphthyl, tetrahydronaphthyl, decanhydronaphthyl, pyridyl, quinolyl, guanidino, pyrrolidyl, pyrrolyl, morpholinyl, furyl, furfuryl, tetrahydrofurfuryl, benzimidazolyl, thienyl, imidazoyl. Preferred substituents on the aryl, cycloalkyl, fused aryl aryl, fused arylcycloalkyl and polycyclic ring structures include hydroxy, alkyl, alkoxy, aryl, aryloxy, aralkyl, alkylamino, dialkylamino, alkenyl, alkynyl, carboxy, carboalkoxy, cyano, mercapto, amino, alkylmercapto, halo, trifluoromethyl, sulfonamide.

The halo groups include fluoro, chloro, bromo, and iodo. Preferred hetero atoms are S, O, and N.

Substituents which are "unsaturated" contain one or more double or triple bonds.

Compounds in accordance with the present invention are readily prepared employing known starting

materials and procedures. It will be understood by those skilled in the art that the carbons to which $R_1$ and $R_3$ are attached can be asymmetric centers, such that the inventive compounds may exist in SS, SR, RS, and RR forms. Individual isomers and diastereoisomeric mixtures of said forms are within the scope of the invention. The preferred forms have (S, S) configuration.

The compounds of the formula (I) can be prepared by reacting a compound of the formula (2):

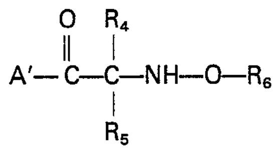
(2)

wherein $R_4$, $R_5$, $R_6$ and A' are defined hereinabove, under amide-forming conditions, with an acylating derivation of an acid of the formula (3):

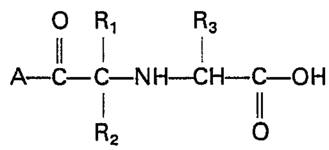
(3)

wherein $R_1$, $R_2$, $R_3$ and A are defined hereinabove; and optionally by substitution and conversion reactions introducing various substituents into the said products; and optionally forming salts thereof, especially pharmaceutically acceptable salts with acids and bases.

A preferred method involves acylation of a formula (2) compound with a compound of formula (4):

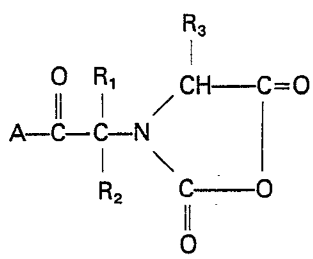
(4)

in which A, $R_1$, $R_2$, and $R_3$ are as defined above, to form a compound (5):

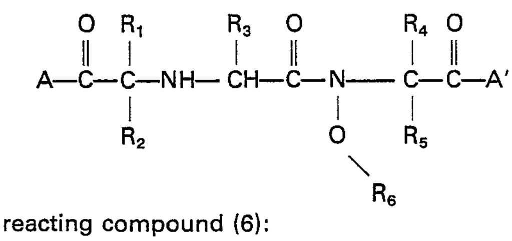
(5)

Compound (4) is obtained by reacting compound (6):

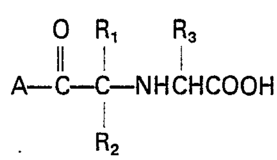
(6)

with an excess of phosgene in methylene chloride with heat and reflux.

As an alternate route to proceedings via a compound (4), one can react compound (6) with, e.g., isobutylene in dioxane to form a compound (7):

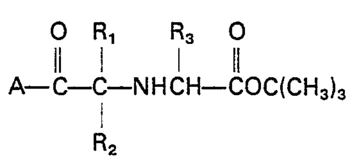
(7)

and then protecting the nitrogen by reaction with a suitable group such as 2,2,2-trichloroethyl chloroformate (8) in pyridine

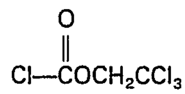

(8)

to form the protected ester (9):

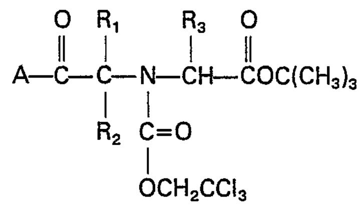

(9)

The ester group on compound (9) is converted to a carboxylic acid group by reaction with strong HCl in dioxane, then converted to the acid chloride by reaction with oxalyl chloride in methylene chloride. The resulting compound (10):

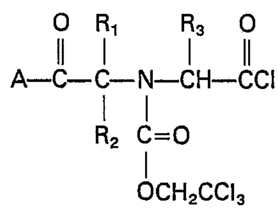

(10)

is reacted with compound (2) and the N-protecting group is removed with, e.g., zinc dust in glacial acetic acid to form compound (5). Each of the above reactions proceeds in a straight-forward manner in a suitable solvent at temperatures ranging from 0°C to 150°C.

The amide forming conditions referred to herein involve the use of known derivatives of the described acids, such as the acyl halides, anhydrides, mixed anhydrides, lower alkyl esters, carbodiimides, carbonyl diimidazoles, and the like. The reactions are carried out in organic solvents such as acetonitrile, tetrahydrofuran, dioxane, acetic acid, methylene chloride, ethylene chloride and similar such solvents. The amide forming reaction will occur at room temperature or at elevated temperature. The use of elevated temperature is for convenience in that it permits somewhat shortened reaction periods. Temperatures ranging from 0°C. up to the reflux temperature of the reaction system can be used. As a further convenience the amide forming reaction can be effected in the presence of a base such as tertiary organic amines, e.g., trimethylamine, pyridine, picolines and the like, particularly where hydrogen halide is formed by the amide-forming reaction, e.g., acyl halide and amino compound. Of course, in those reactions where hydrogen halide is produced, any of commonly used hydrogen halide acceptors can also be used.

Various substituents on the present new compounds e.g., as defined for $R_5$, can be present in the starting compounds or added after formation of the amide products by the known methods of substitution or conversion reactions. Thus, the nitro group can be added to the final product by nitration of the aromatic ring and the nitro group converted to other groups, such as amino by reduction, and halo by diazotization of the amino groups and replacement of the diazo group. Other reactions can be effected on the formed amide product. Amino groups can be alkylated to form mono and dialkylamino groups, mercapto and hydroxy groups can be alkylated to form corresponding ethers. Thus, substitution or alteration reactions can be employed to provide a variety of substituents throughout the molecule of the final products. Of course, reactive groups where present should be protected by suitable blocking groups during any of the aforesaid reactions particularly the condensation reactions to form the amide linkages.

The acid and base salts of the present new compounds can be formed using standard procedures. Often, they are formed *in situ* during the preparation of the present new amido amino acids.

The present compounds obviously exist in stereoisomeric forms and the products obtained thus can be mixtures of the isomers, which can be resolved. Alternatively, by selection of specific isomers as starting compounds, the preferred stereoisomer can be produced. Therefore, the preferred forms, where each asymmetric centrail (chairal center) is S-configuration, are preferably prepared by the stereospecific route rather than attempting resolution of mixtures of isomers. The compounds in which the S-configuration exists at all asymmetric centers are the most active; those in which the R-configuration exists are of less activity; and those where both R- and S-configurations exist are of intermediate activity.

The products are obtained typically as a mixture of diastereoisomers which can be separated by standard methods of fractional crystallization or chromatography.

The compounds of this invention form acid salts with various inorganic and organic acids which are also within the scope of the invention. The pharmaceutically-acceptable acid addition salts of the compounds of the present invention may be prepared by conventional reactions by reacting the free amino acid or amino ester with an appropriate acid providing the desired anion, either in a solvent or medium in

which the salt is insoluble, or in water and removing the water by freeze-drying. The salts of strong acids are preferred. As exemplary, but not limiting, of pharmaceutically-acceptable acid salts are the salts of hydrochloric, hydrobromic, sulfuric, nitric, acetic, furmaric, malic, maleic and citric acids.

Suitable basic salts may include the salts of alkali and alkali earth metals such as sodium, lithium, potassium, magnesium and calcium, as well as iron and salts of ammonia and amines, and quaternary ammonium salts.

The action of the enzyme renin or angiotensinogen, a pseudoglobulin in blood plasma, produces the decapeptide angiotensin I. Angiotensin I is converted by angiotensin converting enzyme (ACE) to the octapeptide angiotensin II. The latter is an active pressor substance which has been implicated as the causative agent in various forms of hypertension in various mammalian species, e.g., rats and dogs. The compounds of this invention intervene in the renin — to — angiotensin I — to — angiotensin II sequence by inhibiting angiotensin I converting enzyme and reducing or eliminating the formation of the pressor substance angiotensin II and therefore are useful in reducing or relieving hypertension. Thus by the administration of a composition containing one or a combination of compounds of formula (1) or pharmaceutically-acceptable salts thereof, hypertension in the species of mammal suffering therefrom is alleviated. A single dose, or preferably two to four divided daily doses, provided on a basis of 0.1 to 100 mg per kilogram per day, preferably 1 to 50 mg per kilogram per day, is appropriate to reduce blood pressure. The substance is preferably administered orally, but a parenteral route such as subcutaneously, intramuscularly, intraveneously or intraperitonealy can also be employed.

The compounds of the present invention exhibit surprising and pronounced antihypertensive activity. When evaluated according to standard, recognized *in vivo* methods, a compound of the present invention in the (S, S) form and having the structure (10):

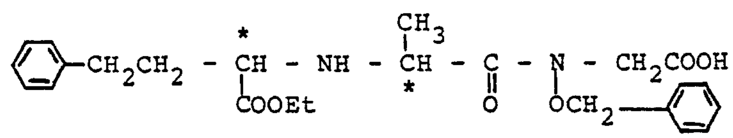

(10)

exhibited an angiotensin converting enzyme inhibition $ED_{50}$ value of less than 1 mg/kg.

The compounds of the invention can be utilized to achieve the reduction of blood pressure by formulating one or more of them in compositions such as tablets, capsules or elixirs for oral administration or in sterile solutions or suspensions for perenteral administration. 10 to 500 mg of a compound or mixture of compounds of formula (1) or physiologically acceptable salt(s) thereof is compounded with a physiologically acceptable vehicle, carrier, excipient, binder, preservative, stabilizer, flavor, etc., in a unit dosage form as called for by accepted pharmaceutical practice. The amount of active substance in these compositions or preparations is such that a suitable dosage in the range indicated is obtained.

Illustrative of the adjuvants which may be incorporated in tablets, capsules and the like are the following: a binder such as gum tragacanth, acacia, corn starch or gelatin; an excipient such as dicalcium phosphate; a disintegrating agent such as corn starch, potato starch, alginic acid and the like, a lubricant such as magnesium stearate; a sweetening agent such as sucrose, lactose or saccharin; a flavoring agent such as peppermint, oil of wintergreen or cherry. When the dosage unit form is a capsule, it may contain in addition to materials of the above type a liquid carrier such as fatty oil. Various other materials may be present as coatings or to otherwise modify the physical form of the dosage unit. For instance, tablets may be coated with shellac, sugar or both. A syrup or elixir may contain the active compound, sucrose as a sweetening agent, methyl and propyl parabens as preservatives, a dye and a flavoring such as cherry or orange flavor.

Sterile compositions for injection can be formulated according to conventional pharmaceutical practice by dissolving or suspending the active substance in a vehicle such as water for injection, a naturally occurring vegetable oil like sesame oil, coconut oil, peanut oil, cottonseed oil, etc., or a synthetic fatty vehicle like ethyl oleate, and the like. Buffers, preservatives, antioxidants and the like can be incorporated as required.

Specific embodiments of the invention are illustrated in the following Examples.

## Example 1

A mixture of 1.6 g of O-benzylhydroxylamine, 2.1 g of t-butylbromoacetate and 1.4 g of $K_2CO_3$ in 10 ml of dimethyl formamide (DMF) was stirred overnight at room temperature. DMF was then removed *in vacuo* and the residue was extracted with ethyl acetate. The organic solution was evaporated to give 2 g of oily product, t-butyl-O-benzyl-α-hydroxylaminoacetic acetate,

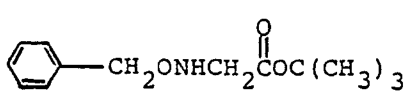

(1-A)

A mixture of N[(1S)-1-ethoxycarbonyl-3-phenylpropyl]-L-alanine (2 g) and an excess of phosgene in 30 ml of methylene chloride was heated to reflux for 2 hours. Evaporation of solvent gave 2.1 g of N-[1-(S)-ethoxycarbonyl-3-phenylpropyl]-S-alanyl-N-carboxyanhydride. A mixture of 1.6 g of this anhydride and 2.1 g of product (I—A) in 10 ml of methylene chloride was stirred overnight at room temperature. The organic solution was then washed with $NaHCO_3$ solution with water, and then dried and evaporated to dryness. Purification by dry column chromatography gave 1.1 g of oil product (I—B), O-benzyl-N-[N-[(1S)-1-ethoxycarbonyl-3-phenylpropyl]-L-alanyl]-α-[hydroxylamino]-acetic acid t-butyl ester,

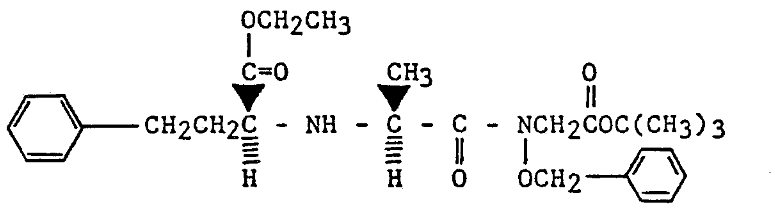

(I-B)

A solution of 1.1 g of product I—B in 100 ml of ether was bubbled with dry HCl gas at 0°C for 2 hours. Evaporation of the ether gave 1.0 g of the hydrochloric acid salt (I—C), O-benzyl-N-[N-[(1S)-1-ethoxycarbonyl-3-phenylpropyl]-L-alanayl]-α [hydroxylamino]-acetic acid HCl salt, a white powder with a melting point of 72—82°C (dec.).

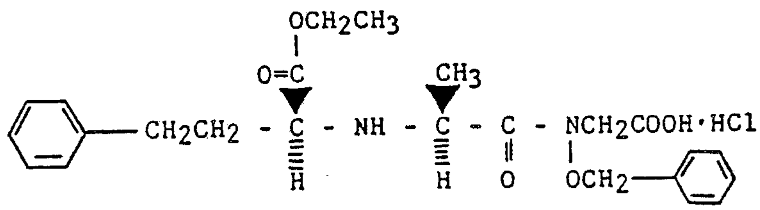

(I-C)

Example II

The following compounds were prepared in a manner wholly analogous to that described in Example I:

(II—A) O-Methyl-N-[N-[(1S)-1-ethoxycarbonyl-3-phenylpropyl]-L-alanyl]-α-[hydroxylamino]-acetic acid t-butyl ester

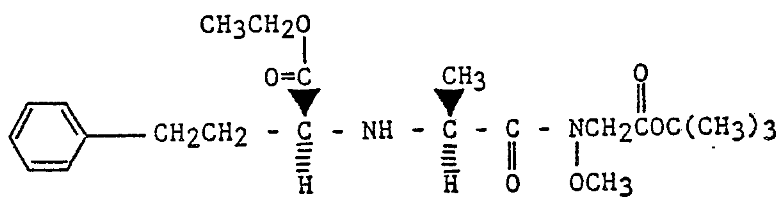

(II-A)

(II—B) O-Methyl-N-[N-[(1S)-1-ethoxycarbonyl-3-phenylpropyl]-L-alanyl]-α-[hydroxylamino]-acetic acid, hydrochloric acid salt

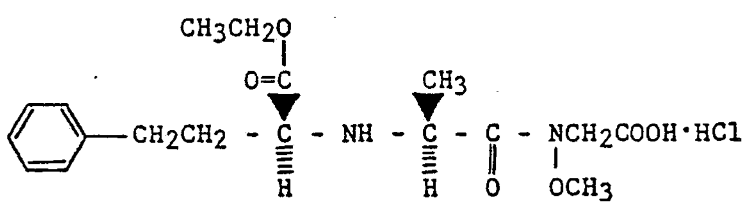

(II-B)

Example III

The following compounds are prepared in a manner wholly analogous to that described in Example I:

(III—A) O-Benzyl-N-[N-[(1S)-1-ethoxycarbonyl-3-phenylpropyl]-L-lysinyl]-α-[hydroxylamino]-acetic acid

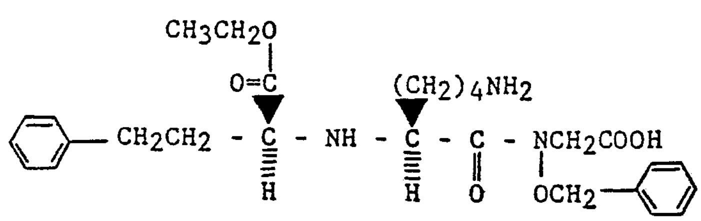

(III—B) O-Benzyl-N-[N-[(1S)-1-ethoxycarbonylethyl]-L-alanyl]-α-[hydroxylamino]-acetic acid

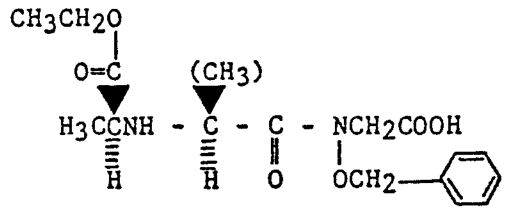

(III—C) O-(4-Pyridylmethyl)-N-[N-[(1S)-1-ethoxycarbonyl-3-phenylpropyl]-L-alanyl]-α-[hydroxylamino]-acetic acid

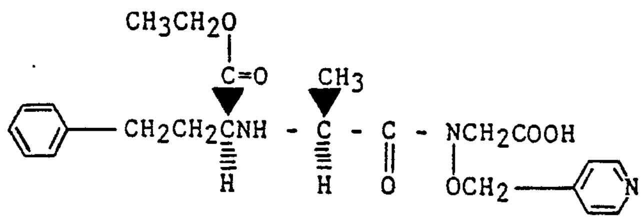

(III—D) O-(Ethoxyethyl)-N-[N-[(1S)-1-ethoxycarbonyl-3-phenylpropyl]-L-alanyl]-α-[hydroxylamino]-acetic acid

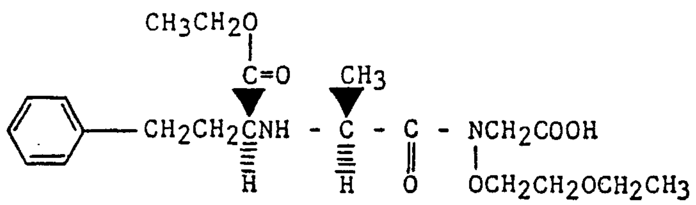

(III—E) O-(Benzyl)-N-[N-[(1S)-1-ethoxycarbonyl-3-phenoxypropyl]-L-alanyl]-α-[hydroxylamino]-acetic acid

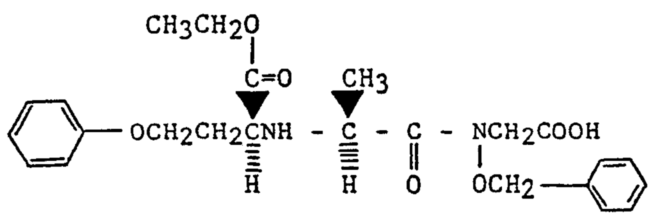

(III—F) O-Benzyl-N-[N-[(1S)-1-ethoxycarbonyl-3-phenylpropyl]-L-alanyl]-α-[hydroxylamino]-propionic acid

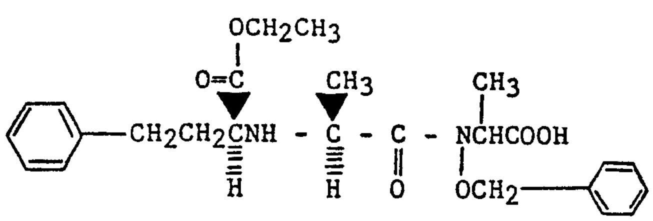

(III—G) O-(1-Naphthylmethyl-N-[N-[(1S)-1-ethoxycarbonyl-3-phenylpropyl]-L-alanyl]-α-[hydroxylamino]-acetic acid

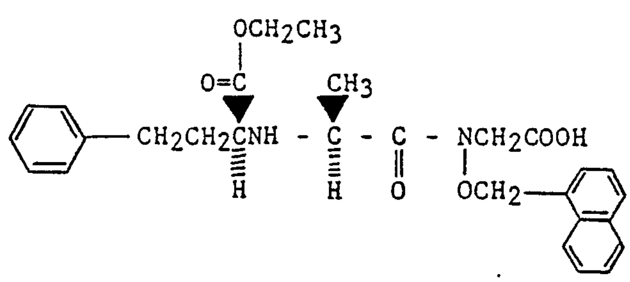

(III—H) O-[N-Morpholinoethyl]-N-[N-[(1S)-ethoxycarbonyl-3-phenylpropyl]-L-alanyl]-α-[hydroxylamino]-acetic acid

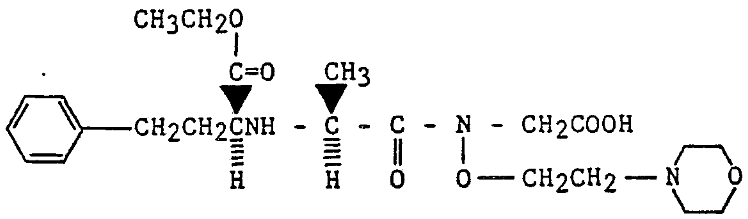

**Claims for the Contracting States: BE CH DE FR GB IT LI LU NL SE**

1. Compounds of the formula

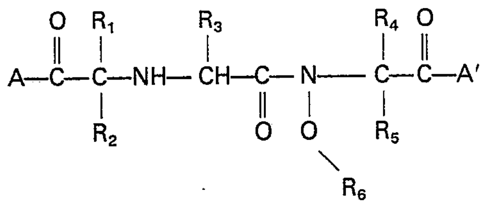

(1)

and their pharmaceutically-acceptable salts, wherein

A and A′ are independently hydroxy, alkoxy, aryloxy, or hydroxyamino;

$R_1$, $R_2$, $R_4$ and $R_5$ are independently hydrogen, alkyl, aryl, aralkyl, fused cycloalkylaryl, fused arylcycloalkyl, aryloxyalkyl, or arylalkyloxyalkyl;

$R_3$ is hydrogen, lower alkyl, lower alkenyl, lower alkynyl, aryl, fused cycloalkylaryl, fused arylcycloalkyl, fused cycloalkylarylalkyl, fused arylcycloalkylalkyl, aralkyl, cycloalkyl, or heterocyclic; and

$R_6$ is hydrogen, alkyl, alkenyl, alkynyl, acyl, cycloalkyl, cycloalkylalkyl, polycycloalkyl, polycycloalkylalkyl, aryl, aralkyl, polycyclic aryl, fused cycloalkylaryl, fused arylcycloalkyl, fused arylcycloalkylalkyl, or fused cycloalkylarylalkyl;

wherein the alkyl, alkoxy, alkenoxy, alkenyl, and alkynyl groups may be substituted with hydroxy, acyloxy, aryl, alkoxy, aryloxy, amino, mono- or dialkylamino, acylamino, mercapto, mercaptoalkyl, or alkylthio; the aryl and cycloalkyl rings may contain one or more hetero atoms, and may be substituted with carboxylic acid, cyano, carbo-lower alkoxy, alkyl, hydroxy, alkoxy, hydroxyalkyl, halo, haloalkyl, mercapto, mercaptoalkyl, alkylthio, thioalkyl, amino, alkylamino, aminoalkyl, nitro, methylenedioxy, or sulfamyl groups and the cycloalkyl groups may be saturated or unsaturated.

2. Compounds according to claim 1 and salts thereof wherein at least one of $R_1$, $R_2$, $R_3$, $R_4$ and $R_5$ is hydrogen.

3. Compounds and salts of claim 1 wherein A and A′ are independently hydroxy or alkoxy; $R_1$, $R_2$, $R_3$, $R_4$ and $R_5$ are independently hydrogen, alkyl, aryl, aralkyl, aryloxyalkyl, or w-aminoalkyl; and $R_6$ is hydrogen, alkyl, aryl, aralkyl, cycloalkyl, alkoxyalkyl, aminoalkyl, heteroaryl, heteroarylalkyl or fused arylaryl.

4. The product according to any of claims 1 to 3 which is one of the following:

O-benzyl-N-[N-[(1S)-1-ethoxycarbonyl-3-phenylpropyl]-L-alanyl]-α-[hydroxylamino]-acetic acid

O-benzyl-N-[N-[(1S)-1-ethoxycarbonyl-3-phenylpropyl]-L-alanyl]-α-[hydroxylamino]-acetic acid t-butyl ester

O-methyl-N-[N-[(1S)-1-ethoxycarbonyl-3-phenylpropyl]-L-alanyl]-α-[hydroxylamino]-acetic acid

O-methyl-N-[N-[(1S)-1-ethoxycarbonyl-3-phenylpropyl]-L-alanyl]-α-[hydroxylamino]-acetic acid t-butyl ester

O-benzyl-N-[N-[(1S)-1-ethoxycarbonyl-3-phenylpropyl]-L-lysinyl]-α-[hydroxylamino]-acetic acid

O-benzyl-N-[N-[(1S)-1-ethoxycarbonylethyl]-L-alanyl]-α-[hydroxylamino]-acetic acid

O-(4-pyridylmethyl)-N-[N-[(1S)-1-ethoxycarbonyl-3-phenylpropyl]-L-alanyl]-α-[hydroxylamino]-acetic acid

O-(ethoxyethyl)-N-[N-[(1S)-1-ethoxycarbonyl-3-phenylpropyl]-L-alanyl]-α-[hydroxylamino]-acetic acid

O-benzyl-N-[N-[(1S)-1-ethoxycarbonyl-3-phenoxypropyl]-L-alanyl]-α-[hydroxylamino]-acetic acid

O-benzyl-N-[N-[(1S)-1-ethoxycarbonyl-3-phenylpropyl]-L-alanyl]-α-[hydroxylamino]-propionic acid

O-(1-Naphthylmethyl-N-[N-[(1S)-1-ethoxycarbonyl-3-phenylpropyl]-L-alanyl]-α-[hydroxylamino]-acetic acid

O-[N-Morpholinoethyl]-N-[N-[(1S)-1-ethoxycarbonyl-3-phenylpropyl]-L-alanyl]-α-[hydroxylamino]-acetic acid.

5. A pharmaceutical preparation comprising one or more components according to claims 1 to 4, in association with a pharmaceutically-acceptable carrier.

6. A process for producing a compound of formula (1) according to claim 1 which comprises reacting under amide forming conditions an amino compound of formula (II)

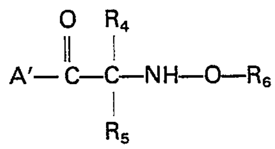

(II)

with an acylating derivative of an acid of formula (III)

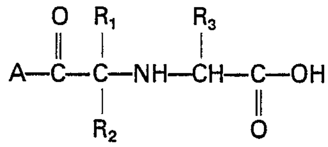

(III)

and optionally by substitution or conversion reactions introducing various substituents into the products;

and optionally forming salts thereof, especially pharmaceutically-acceptable salts with acids and bases.

**Claims for the Contracting State: AT**

1. A process for producing a compound of formula (I)

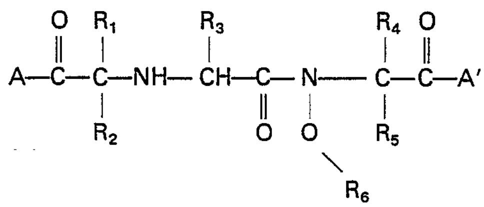

(I)

and their pharmaceutically-acceptable salts,

wherein A and A′ are independently hydroxy, alkoxy, aryloxy, or hydroxyamino;

$R_1$, $R_2$, $R_4$ and $R_5$ are independently hydrogen, alkyl, aryl, aralkyl, fused cycloalkylaryl, fused arylcycloalkyl, aryloxyalkyl, or arylalkyloxyalkyl;

$R_3$ is hydrogen, lower alkyl, lower alkenyl, lower alkynyl, aryl, fused cycloalkylaryl, fused arylcycloalkyl, fused cycloalkylarylalkyl, fused arylcycloalkylalkyl, aralkyl, cycloalkyl, or heterocyclic; and

$R_6$ is hydrogen, alkyl, alkenyl, alkynyl, acyl, cycloalkyl, cycloalkylalkyl, polycycloalkyl, polycycloalkylalkyl, aryl, aralkyl, polycyclic aryl, fused cycloalkylaryl, fused arylcycloalkyl, fused arylcycloalkylalkyl, or fused cycloalkylarylalkyl;

wherein the alkyl, alkoxy, alkenoxy, alkenyl, and alkynyl groups may be substituted with hydroxy, acyloxy, aryl, alkoxy, aryloxy, amino, mono- or dialkylamino, acylamino, mercapto, mercaptoalkyl, or alkylthio; the aryl and cycloalkyl rings may contain one or more hetero atoms, and may be substituted with carboxylic acid, cyano, carbo-lower alkoxy, alkyl, hydroxy, alkoxy, hydroxyalkyl, halo, haloalkyl, mercapto, mercaptoalkyl, alkylthio, thioalkyl, amino, alkylamino, aminoalkyl, nitro, methylenedioxy, or sulfamyl groups and the cycloalkyl groups may be saturated or unsaturated, which comprises reacting under amide forming conditions an amino compound of formula (II)

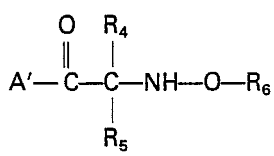

(II)

with an acylating derivative of an acid of formula (III)

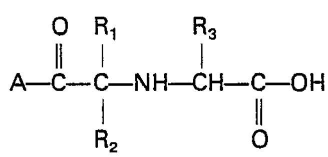

(III)

and optionally by substitution or conversion reactions introducing various substituents into the products;

and optionally forming salts thereof, especially pharmaceutically-acceptable salts with acids and bases.

2. The process according to claim 1 wherein at least one of $R_1$, $R_2$, $R_3$, $R_4$ and $R_5$ is hydrogen.

3. The process according to claim 1 wherein

A and A' are independently hydroxy or alkoxy;

$R_1$, $R_2$, $R_3$, $R_4$ and $R_5$ are independently hydrogen, alkyl, aryl, aralkyl, aryloxyalkyl or w-aminoalkyl; and

$R_6$ is hydrogen, alkyl, aryl, aralkyl, cycloalkyl, alkoxyalkyl, aminoalkyl, heteroaryl, heteroarylalkyl or fused arylaryl.

4. The process according to any of claims 1 to 3 wherein the product is one of the following:

O-benzyl-N-[N-[(1S)-1-ethoxycarbonyl-3-phenylpropyl]-L-alanyl]-α-[hydroxylamino]-acetic acid

O-benzyl-N-[N-[(1S)-1-ethoxycarbonyl-3-phenylpropyl]-L-alanyl]-α-[hydroxylamino]-acetic acid t-butyl ester

O-methyl-N-[N-[(1S)-1-ethoxycarbonyl-3-phenylpropyl]-L-alanyl]-α-[hydroxylamino]-acetic acid

O-methyl-N-[N-[(1S)-1-ethoxycarbonyl-3-phenylpropyl]-L-alanyl]-α-[hydroxylamino]-acetic acid t-butyl ester

O-benzyl-N-[N-[(1S)-1-ethoxycarbonyl-3-phenylpropyl]-L-lysinyl]-α-[hydroxylamino]-acetic acid

O-benzyl-N-[N-[(1S)-1-ethoxycarbonylethyl]-L-alanyl]-α-[hydroxylamino]-acetic acid

O-(4-pyridylmethyl)-N-[N-[(1S)-1-ethoxycarbonyl-3-phenylpropyl]-L-alanyl]-α-[hydroxylamino]-acetic acid

O-(ethoxyethyl)-N-[N-[(1S)-1-ethoxycarbonyl-3-phenylpropyl]-L-alanyl]-α-[hydroxylamino]-acetic acid

O-benzyl-N-[N-[(1S)-1-ethoxycarbonyl-3-phenoxypropyl]-L-alanyl]-α-[hydroxylamino]-acetic acid

O-benzyl-N-[N-[(1S)-1-ethoxycarbonyl-3-phenylpropyl]-L-alanyl]-α-[hydroxylamino]-propionic acid

O-(1-Naphthylmethyl-N-[N-[(1S)-1-ethoxycarbonyl-3-phenylpropyl]-L-alanyl]-α-[hydroxylamino]-acetic acid

O-[N-Morpholinoethyl]-N-[N-[(1S)-1-ethoxycarbonyl-3-phenylpropyl]-L-alanyl]-α-[hydroxylamino]-acetic acid.

5. A process for producing a pharmaceutical preparation comprising formulating at least one compound of formula (1) according to any of claims 1 to 4, with pharmaceutically-acceptable carriers and/or diluents.

**Patentansprüche für die Vertragsstaaten: BE CH DE FR GB IT LI LU NL SE**

1. Verbindungen der Formel

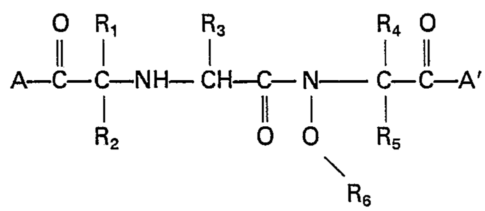

(I)

und ihre pharmazeutisch annehmbaren Salze, worin

A und A' unabhängig voneinander Hydroxy, Alkoxy, Aryloxy oder Hydroxyamino bedeuten;

$R_1$, $R_2$, $R_4$ und $R_5$ unabhängig voneinander Wasserstoff, Alkyl, Aryl, Aralkyl, kondensiertes Cycloalkylaryl, kondensiertes Arylcycloalkyl, Arylyloxyalkyl oder Arylalkoxylalkyl bedeuten,

$R_3$ Wasserstoff, Niedrigalkyl, Niedrigalkenyl, Niedrigalkynyl, Aryl, kondensiertes Cycloalkylaryl, kondensiertes Arylcycloalkyl, kondensiertes Cycloalkylarylalkyl, kondensiertes Arylcycloalkylalkyl, Aralkyl, Cycloalkyl oder einen Heterocyclus bedeutet, und

$R_6$ Wasserstoff, Alkyl, Alkenyl, Alkynyl, Acyl, Cycloalkyl, Cycloalkylalkyl, Polycycloalkyl, Polycycloalkylalkyl, Aryl, Aralkyl, polycyclisches Aryl, kondensiertes Cycloalkylaryl, kondensiertes Arylcycloalkyl, kondensiertes Arylcycloalkylalkyl oder kondensiertes Cycloalkylarylalkyl bedeutet,

worin die Alkyl-, Alkoxy-, Alkenoxy-, Alkenyl- und Alkynylgruppen durch Hydroxy, Acyloxy, Aryl, Alkoxy, Aryloxy, Amino, Mono- oder Dialkylamino, Acrylamino, Mercapto, Mercaptoalkyl oder Alkylthio substituiert sein können; die Aryl- und Cycloalkylringe ein oder mehrere Heteroatome enthalten können und durch Carbonsäure-, Cyano-, Carbo-Niedrigalkoxy-, Alkyl-, Hydroxy-, Alkoxy-, Hydroxyalkyl-, Halogen-, Halogenalkyl-, Mercapto-, Mercaptoalkyl-, Alkylthio-, Thioalkyl-, Amino-, Alkylamino-, Aminoalkyl-, Nitro-, Methylendioxy- oder Sulfamylgruppen substituiert sein können und die Cycloalkylgruppen gesättigt oder ungesättigt sein können.

2. Verbindungen nach Anspruch 1 und ihre Salze, worin wenigstens einer der Substituenten $R_1$, $R_2$, $R_3$, $R_4$ und $R_5$ Wasserstoff ist.

3. Verbindungen und Salze nach Anspruch 1, worin A und A' unabhängig voneinander Hydroxy oder Alkoxy bedeuten, $R_1$, $R_2$, $R_3$, $R_4$ und $R_5$ unabhängig voneinander Hydrogen, Alkyl, Aryl, Aralkyl, Aryloxyalkyl oder ω-Aminoalkyl bedeuten und $R_6$ Wasserstoff, Alkyl, Aryl, Aralkyl, Cycloalkyl, Alkoxyalkyl, Aminoaryl, Heteroaryl, Heteroarylalkyl oder kondensiertes Arylaryl ist.

4. Produkt nach einem der Ansprüche 1 bis 3, welches eines der folgenden ist:

O-Benzyl-N-[N-[(1S)-1-ethoxycarbonyl-3-phenylpropyl]-L-alanyl]-α-[hydroxylamino]-essigsäure

O-Benzyl-N-[N-[(1S)-1-ethoxycarbonyl-3-phenylpropyl]-L-alanyl]-α-[hydroxylamino]-essigsäure-t-butylester

O-Methyl-N-[N-[(1S)-1-ethoxycarbonyl-3-phenylpropyl]-L-alanyl]-α-[hydroxylamino]-essigsäure

O-Methyl-N-[N-[(1S)-1-ethoxycarbonyl-3-phenylpropyl]-L-alanyl]-α-[hydroxylamino]-essigsäure-t-butylester

O-Benzyl-N-[N-[(1S)-1-ethoxycarbonyl-3-phenylpropyl]-L-lysinyl]-α-[hydroxylamino]-essigsäure

O-Benzyl-N-[N-[(1)-1-ethoxycarbonylethyl]-L-alanyl]-α-[hydroxylamino]-essigsäure

O-(4-Pyridylmethyl)-N-[N-[(1S)-1-ethoxycarbonyl-3-phenylpropyl]-L-alanyl]-α-[hydroxylamino]-essigsäure

O-(Ethoxyethyl)-N-[N-[(1S)-1-ethoxycarbonyl-3-phenylpropyl]-L-alanyl]-α-[hydroxylamino]-essigsäure

O-Benzyl-N-[N-[(1S)-1-ethoxycarbonyl-3-phenoxypropyl]-L-alanyl]-α-[hydroxylamino]-essigsäure

O-Benzyl-N-[N-[(1S)-1-ethoxycarbonyl-3-phenylpropyl]-L-alanyl]-α-[hydroxylamino]-propionsäure

O-(1-Naphthylmethyl-N-[N-[(1S)-1-ethoxycarbonyl-3-phenylpropyl]-L-alanyl]-α-[hydroxylamino]-essigsäure

O-[N-Morpholinoethyl]-N-[N-[(1S)-1-ethoxycarbonyl-3-phenylpropyl]-L-alanyl]-α-[hydroxylamino]-essigsäure.

5. Pharmazeutische Herstellung, umfassend eine oder mehrere Komponenten nach den Ansprüchen 1 bis 4, in Verbindung mit einem pharmazeutisch annehmbaren Träger.

6. Verfafhren zur Herstellung einer Verbindung der Formel 1 nach Anspruch 1, welches die Umsetzung unter amidbildenden Bedingungen einer Aminoverbindung der Formel II

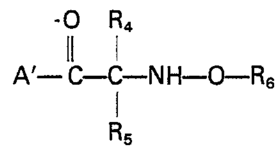

(II)

mit einem acylierenden Derivat einer Säure der Formel (III)

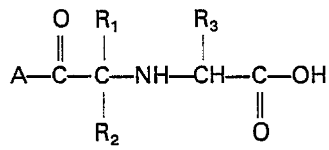

(III)

und gegebenenfalls das Einführen verschiedener Substituenten in die Produkte durch Substitutions- oder Umwandlungsreaktionen und gegebenenfalls die Bildung ihrer Salze, insbesondere ihrer pharmazeutisch annehmbaren Salze mit Säuren und Basen, umfaßt.

**Patentansprüche für den Vertragsstaat: AT**

1. Verfahren zur Herstellung einer Verbindung der Formel I

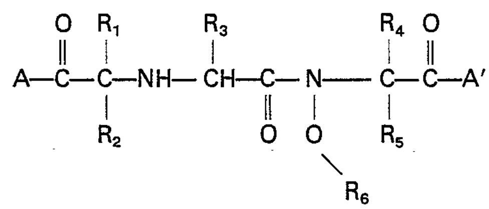

(I)

und ihre pharmazeutisch annehmbaren Salze, worin

A und A' unabhängig voneinander Hydroxy, Alkoxy, Aryloxy oder Hydroxyamino bedeuten;

$R_1$, $R_2$, $R_4$ und $R_5$ unabhängig voneinander Wasserstoff, Alkyl, Aryl, Aralkyl, kondensiertes Cycloalkylaryl, kondensiertes Arylcycloalkyl, Aryloxyalkyl oder Arylalkyloxyalkyl bedeuten,

$R_3$ Wasserstoff, Niedrigalkyl, Niedrigalkenyl, Niedrigalkynyl, Aryl, kondensiertes Cycloalkylaryl, kondensiertes Arylcycloalkyl, kondensiertes Cycloalkylarylalkyl, kondensiertes Arylcycloalkylalkyl, Aralkyl, Cycloalkyl oder einen Heterocyclus bedeutet, und

$R_6$ Wasserstoff, Alkyl, Alkenyl, Alkynyl, Acyl, Cycloalkyl, Cycloalkylalkyl, Polycycloalkyl, Polycycloalkylalkyl, Aryl, Aralkyl, polycyclisches Aryl, kondensiertes Cycloalkylaryl, kondensiertes Arylcycloalkyl, kondensiertes Arylcycloalkylalkyl oder kondensiertes Cycloalkylarylalkyl bedeutet,

worin die Alkyl-, Alkoxy-, Alkenoxy-, Alkenyl- und Alkynylgruppen durch Hydroxy, Acyloxy, Aryl, Alkoxy, Aryloxy, Amino, Mono- oder Dialkylamino, Acrylamino, Mercapto, Mercaptoalkyl oder Alkylthio substituiert sein können; die Aryl- und Cycloalkylringe ein oder mehrere Heteroatome enthalten können und durch Carbonsäure-, Cyano-, Carbo-Niedrigalkoxy-, Alkyl-, Hydroxy-, Alkoxy-, Hydroxyalkyl-, Halogen-,

Halogenalkyl-, Mercapto-, Mercaptoalkyl-, Alkylthio-, Thioalkyl-, Amino-, Alkylamino-, Aminoalkyl-, Nitro-, Methylendioxy- oder Sulfamylgruppen substituiert sein können und die Cycloalkylgruppen gesättigt oder ungesättigt sein können, welches die Umsetzung unter amidbildenden Bedingungen einer Aminoverbindung der Formel II

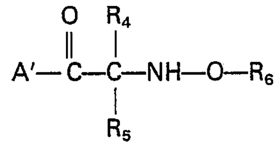

(II)

mit einem acylierenden Derivat einer Säure der Formel III

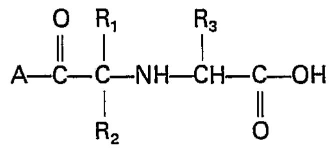

(III)

und gegebenenfalls das Einführen verschiedener Substituenten in die Produkte durch Substitutions- oder Umwandlungsreaktionen und gegebenenfalls die Bildung ihrer Salze, insbesondere ihrer pharmazeutisch annehmbaren Salze mit Säuren und Basen, umfaßt.

2. Verfahren nach Anspruch 1, worin wenigstens einer der Substituenten $R_1$, $R_2$, $R_3$, $R_4$ und $R_5$ Wasserstoff ist.

3. Verfahren nach Anspruch 1, worin A und A' unabhängig voneinander Hydroxy oder Alkoxy bedeuten, $R_1$, $R_2$, $R_3$, $R_4$ und $R_5$ unabhängig voneinander Hydrogen, Alkyl, Aryl, Aralkyl, Aryloxyalkyl oder ω-Aminoalkyl bedeuten und $R_6$ Wasserstoff, Alkyl, Aryl, Aralkyl, Cycloalkyl, Alkoxyalkyl, Aminoalkyl, Heteroaryl, Heteroarylalkyl oder kondensiertes Arylaryl ist.

4. Verfahren nach einem der Ansprüche 1 bis 3, worin das Produkt eines der folgenden ist:

O-Benzyl-N-[N-[(1S)-1-ethoxycarbonyl-3-phenylpropyl]-L-alanyl]-α-[hydroxylamino]-essigsäure

O-Benzyl-N-[N-[(1S)-1-ethoxycarbonyl-3-phenylpropyl]-L-alanyl]-α-[hydroxylamino]-essigsäure-t-butylester

O-Methyl-N-[N-[(1S)-1-ethoxycarbonyl-3-phenylpropyl]-L-alanyl]-α-[hydroxylamino]-essigsäure

O-Methyl-N-[N-[(1S)-1-ethoxycarbonyl-3-phenylpropyl]-L-alanyl]-α-[hydroxylamino]-essigsäure-t-butylester

O-Benzyl-N-[N-[(1S)-1-ethoxycarbonyl-3-phenylpropyl]-L-lysinyl]-α-[hydroxylamino]-essigsäure

O-Benzyl-N-[N-[(1S)-1-ethoxycarbonylethyl]-L-alanyl]-α-[hydroxylamino]-essigsäure

O-(4-Pyridylmethyl)-N-[N-[(1S)-1-ethoxycarbonyl-3-phenylpropyl]-L-alanyl]-α-[hydroxylamino]-essigsäure

O-(Ethoxyethyl)-N-[N-[(1S)-1-ethoxycarbonyl-3-phenylpropyl]-L-alanyl]-α-[hydroxylamino]-essigsäure

O-Benzyl-N-[N-[(1S)-1-ethoxycarbonyl-3-phenoxypropyl]-L-alanyl]-α-[hydroxylamino]-essigsäure

O-Benzyl-N-[N-[(1S)-1-ethoxycarbonyl-3-phenylpropyl]-L-alanyl]-α-[hydroxylamino]-propionsäure

O-(1-Naphthylmethyl-N-[N-[(1S)-1-ethoxycarbonyl-3-phenylpropyl]-L-alanyl]-α-[hydroxylamino]-essigsäure

O-[N-Morpholinoethyl]-N-[N-[(1S)-1-ethoxycarbonyl-3-phenylpropyl]-L-alanyl]-α-[hydroxylamino]-essigsäure.

5. Verfahren zur Herstellung einer pharmazeutischen Herstellung, umfassend die Formulierung wenigstens einer Verbindung der Formel 1 nach einem der Ansprüche 1 bis 4 mit pharmazeutisch annehmbaren Trägern und/oder Verdünnungsmitteln.

**Revendications pour les Etats contractants: BE CH DE FR GB IT LI LU NL SE**

1. Composés de formule:

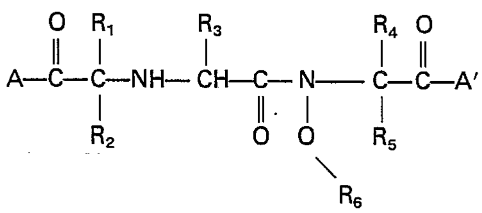

(I)

et leurs sels pharmaceutiquement acceptables, formule dans laquelle:

— A et A' représentent, indépendamment, un groupe hydroxy, alcoxy, aryloxy ou hydroxyamino;

— $R_1$, $R_2$, $R_4$ et $R_5$ représentent, indépendamment, un atome d'hydrogène, un groupe alkyle, aryle, aralkyle, cycloalkylaryle condensé, arylcycloalkyle condensé, aryloxyalkyle ou arylalkyloxyalkyle;

— $R_3$ représente un atome d'hydrogène, un groupe alkyle inférieur, alcényle inférieur, alcynyle inférieur, aryle, cycloalkylaryle condensé, arylcycloalkyle condensé, cycloalkylarylalkyle condensé, arylcycloalkylalkyle condensé, aralkyle, cycloalkyle ou hétérocyclique; et

— $R_6$ représente l'hydrogène, un groupe alkyle, alcényle, alcynyle, acyle, cycloalkyle, cycloalkylalkyle, polycycloalkyle, polycycloalkylalkyle, aryle, aralkyle, aryle polycyclique, cycloalkylaryle condensé, arylcycloalkyle condensé, arylcycloalkylalkyle condensé ou cycloalkylarylalkyle condensé; où les groupes alkyle, alcoxy, alcénoxy, alcényle et alcynyle peuvent être substitués par des groupes hydroxy, acyloxy, aryle, alcoxy, aryloxy, amino, mono- ou dialkylamino, acylamino, mercapto, mercaptoalkyle ou alkylthio; les noyaux aryle et cycloalkyle peuvent contenir un ou plusieurs hétéroatomes, et peuvent être substitués par un reste acide carboxylique, cyano, carbo-alcoxy inférieur, alkyle, hydroxy, alcoxy, hydroxyalkyle, halo, haloalkyle, mercapto, mercaptoalkyle, alkylthio, thioalkyle, amino, alkylamino, aminoalkyle, nitro, méthylènedioxy ou sulfamyle et les groupes cycloalkyle peuvent être saturés ou intarurés.

2. Composés selon la revendication 1 et leurs sels, dans lesquels au moins l'un parmi $R_1$, $R_2$, $R_3$, $R_4$ et $R_5$ représente un atome d'hydrogène.

3. Composés et sels selon la revendication 1, dans lesquels A et A' représentent, indépendamment, un reste hydroxy ou alcoxy; $R_1$, $R_2$, $R_3$, $R_4$ et $R_5$ représentent, indépendamment, un atome d'hydrogène, un reste alkyle, aryle, aralkyle, aryloxyalkyle ou ω-aminoalkyle; et $R_6$ représente un atome d'hydrogène, un reste alkyle, aryle, aralkyle, cycloalkyle, alcoxyalkyle, aminoalkyle, hétéroaryle, hétéroarylalkyle ou arylaryle condensé.

4. Produit selon l'une des revendications 1 à 3 qui est l'un des suivants:

— acide O-benzyl-N-[N-[(1S)-éthoxycarbonyl-1 phényl-3 propyl]-L-lysinyl]-α-[hydroxylamino]-acétique;

— ester t-butylique de l'acide O-benzyl-N-[N-[(1S)-éthoxycarbonyl-1 phenyl-3 propyl]-L-alanyl]-α-[hydroxylamino]-acétique;

— acide O-méthyl-N-[N-[(1S)-ethoxycarbonyl-1 phényl-3 propyl]-L-lysinyl]-α-[hydroxylamino]-acétique;

— ester t-butylique de l'acide O-méthyl-N-[N-[(1S)-éthoxycarbonyl-1 phényl-3 propyl]-L-alanyl]-α-[hydroxylamino]-acétique;

— acide O-benzyl-N-[N-[(1S)-éthoxycarbonyl-1 phényl-3 propyl]-L-lysinyl]-α-[hydroxylamino]-acétique;

— acide O-benzyl-N-[N-[(1S)-éthoxycarbonyl-1 éthyl]-L-alanyl]-α-[hydroxylamino]-acétique;

— acide O-(pyridyl-4 mèthyl)-N-[N-[(1S)-éthoxycarbonyl-1 phényl-3 propyl]-L-alanyl]-α-[hydroxyl-amino]-acétique;

— acide O-(éthoxyéthyl)-N-[N-[(1S)-éthoxycarbonyl-1 phényl-3 propyl]-L-alanyl]-α-[hydroxylamino]-acétique;

— acide O-benzyl-N-[N-[(1S)-éthoxycarbonyl-1 phénoxy-3 propyl]-L-alanyl]-α-[hydroxylamino]-acétique;

— acide O-benzyl-N-[N-[(1S)-éthoxycarbonyl-1 phényl-3 propyl]-L-alanyl]-α-[hydroxylamino]-propionique;

— acide O-(naphthyl-1 méthyl)-N-[N-[(1S)-éthoxycarbonyl-1 phényl-3 propyl]-L-alanyl]-α-[hydroxyl-amino]-acétique;

— acide O-[N-morpholinoéthyl]-N-[N-[(1S)-éthoxycarbonyl-1 phényl-3 propyl]-L-alanyl]-α-[hydroxyl-amino]-acétique.

5. Préparation pharmaceutique comprenant un ou plusieurs composants selon les revendications 1 à 4, en association avec un support pharmaceutiquement acceptable.

6. Procédé de préparation d'un composé de formule 1 selon la revendication 1, qui consiste à faire réagir, dans des conditions de formation d'un amide, un composé amino de formule II:

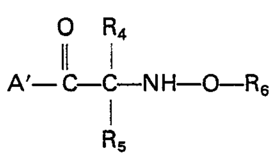

(II)

avec un dérivé acylant d'un acide de formule III:

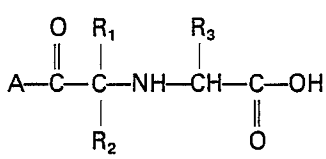

(III)

et, de façon facultative, par des réactions de substitution ou de conversion, à introduire divers substituants dans les produits;

et, de façon facultative, à former des sels de ces derniers, particulièrement des sels pharmaceutiquement acceptables avec des acides et des bases.

**Revendications pour l'Etat contractant: AT**

1. Procédé de préparation d'un composé de formule:

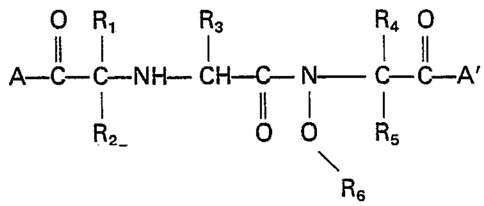

(I)

et leurs sels pharmaceutiquement acceptables, formule dans laquelle:

— A et A' représentent, indépendamment, un groupe hydroxy, alcoxy, aryloxy ou hydroxyamino;

— $R_1$, $R_2$, $R_4$ et $R_5$ représentent, indépendamment, un atome d'hydrogène, un groupe alkyle, aryle, aralkyle, cycloalkylaryle condensé, arylcycloalkyle condensé, aryloxyalkyle ou arylalkyloxyalkyle;

— $R_3$ représente un atome d'hydrogène, un groupe alkyle inférieur, alcényle inférieur, alcynyle inférieur, aryle, cycloalkylaryle condensé, arylcycloalkyle condensé, cycloalkylarylalkyle condensé, arylcycloalkylalkyle condensé, aralkyle, cycloalkyle ou hétérocyclique; et

— $R_6$ représente l'hydrogène, un groupe alkyle, alcényle, alcynyle, acyle, cycloalkyle, cycloalkylalkyle, polycycloalkyle, polycycloalkylalkyle, aryle, aralkyle, aryle polycyclique, cycloalkylaryle condensé, arylcycloalkyle condensé, arylcycloalkylalkyle condensé ou cycloalkylarylalkyle condensé; où les groupes alkyle, alcoxy, alcénoxy, alcényle et alcynyle peuvent être substitués par des groupes hydroxy, acyloxy, aryle, alcoxy, aryloxy, amino, mono- ou dialkylamino, acylamino, mercapto, mercaptoalkyle ou alkylthio; les noyaux aryle et cycloalkyle peuvent contenir un ou plusieurs hétéroatomes, et peuvent être substitués par un reste acide carboxylique, cyano, carbo-alcoxy inférieur, alkyle, hydroxy, alcoxy, hydroxyalkyle, halo, haloalkyle, mercapto, mercaptoalkyle, alkylthio, thioalkyle, amino, alkylamino, aminoalkyle, nitro, méthylènedioxy ou sulfamyle et les groupes cycloalkyle peuvent être saturés ou insaturés, procédé consistant à faire réagir, dans des conditions de formation d'un amide, un composé amino de formule II:

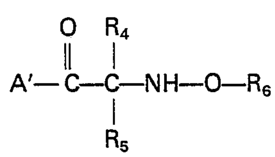

(II)

avec un dérivé acylant d'un acide de formule III:

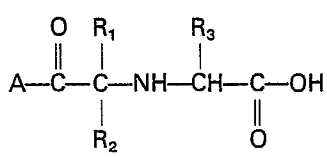

(III)

et, de façon facultative, par des réactions de substitution ou de conversion, à introduire divers substituants dans les produits;

et, de façon facultative, à former des sels de ces derniers, particulièrement des sels pharmaceutiquement acceptables avec des acides et des bases.

2. Procédé selon la revendication 1, dans lesquel au moins l'un parmi $R_1$, $R_2$, $R_3$, $R_4$ et $R_5$ représente un atome d'hydrogène.

3. Procédé selon la revendication 1, dans lesquel A et A' représentent, indépendamment, un reste hydroxy ou alcoxy; $R_1$, $R_2$, $R_3$, $R_4$ et $R_5$ représentent, indépendamment, un atome d'hydrogène, un reste alkyle, aryle, aralkyle, aryloxyalkyle ou ω-aminoalkyle; et $R_6$ représente un atome d'hydrogène, un reste alkyle, aryle, aralkyle, cycloalkyle, alcoxyalkyle, aminoalkyle, hétéroaryle, hétéroarylalkyle ou arylaryle condensé.

4. Procédé selon l'une des revendications 1 à 3, dans lequel le produit est l'un des suivants:

— acide O-benzyl-N-[N-[(1S)-éthoxycarbonyl-1 phényl-3 propyl]-L-alanyl]-α-[hydroxylamino]-acétique;

— ester t-butylique de l'acide O-benzyl-N-[N-[(1S)-éthoxycarbonyl-1 phenyl-3 propyl]-L-alanyl]-α-[hydroxylamino]-acétique;

— acide O-méthyl-N-[N-[(1S)-éthoxycarbonyl-1 phényl-3 propyl]-L-alanyl]-α-[hydroxylamino]-acétique;

— ester t-butylique de l'acide O-méthyl-N-[N-[(1S)-éthoxycarbonyl-1 phényl-3 propyl]-L-alanyl]-α-[hydroxylamino]-acétique;

— acide O-benzyl-N-[N-[(1S)-éthoxycarbonyl-1 phényl-3 propyl]-L-lysinyl]-α-[hydroxylamino]-acétique;

— acide O-benzyl-N-[N-[(1S)-éthoxycarbonyl-1 éthyl]-L-alanyl]-α-[hydroxylamino]-acétique;

— acide O-(pyridyl-4 mèthyl)-N-[N-[(1S)-éthoxycarbonyl-1 phényl-3 propyl]-L-alanyl]-α-[hydroxylamino]-acétique;

— acide O-(éthoxyéthyl)-N-[N-[(1S)-éthoxycarbonyl-1 phényl-3 propyl]-L-alanyl]-α-[hydroxylamino]-acétique;

— acide O-benzyl-N-[N-[(1S)-éthoxycarbonyl-1 phénoxy-3 propyl]-L-alanyl]-α-[hydroxylamino]-acétique;

— acide O-benzyl-N-[N-[(1S)-éthoxycarbonyl-1 phényl-3 propyl]-L-alanyl]-α-[hydroxylamino]-propionique;

— acide O-(naphthyl-1 méthyl)-N-[N-[(1S)-éthoxycarbonyl-1 phényl-3 propyl]-L-alanyl]-α-[hydroxylamino]-acétique;

— acide O-[N-morpholinoéthyl]-N-[N-[(1S)-éthoxycarbonyl-1 phényl-3 propyl]-L-alanyl]-α-[hydroxylamino]-acétique.

5. Procédé d'obtention d'une préparation pharmaceutique consistant à formuler au moins un composé de formule 1 selon l'une des revendications 1 à 4, avec des supports et/ou diluants pharmàceutiquement acceptables.